Europäisches Patentamt

European Patent Office

Office européen des brevets

(19)

(11) Veröffentlichungsnummer: **0 113 877**
**A2**

(12) # EUROPÄISCHE PATENTANMELDUNG

(21) Anmeldenummer: **83112524.0**

(22) Anmeldetag: **13.12.83**

(51) Int. Cl.³: **C 12 M 1/00**

(30) Priorität: **16.12.82 DE 3246590**

(43) Veröffentlichungstag der Anmeldung: **25.07.84**
**Patentblatt 84/30**

(84) Benannte Vertragsstaaten: **AT BE CH FR GB IT LI LU NL SE**

(71) Anmelder: **Neubauer, Josef, Wehrlestrasse 4, D-8000 München 80 (DE)**

(72) Erfinder: **Neubauer, Josef, Wehrlestrasse 4, D-8000 München 80 (DE)**

(74) Vertreter: **Graf, Helmut, Dipl.-Ing., Greflinger Strasse 7 Postfach 382, D-8400 Regensburg (DE)**

(54) **Einrichtung zur Gewinnung von Energie in Form von Biogas oder Rottewärme.**

(57) Die Erfindung bezieht sich auf eine Einrichtung zur Gewinnung von Energie in Form von Biogas oder Rottewärme aus organischen Substanzen, mit einem eine Flüssigkeit aufnehmenden Behälter, mit einem im Innenraum dieses Behälters angeordneten Trommelreaktor, der um seine Trommelachse rotierend angetrieben werden kann und einen von einem Rohrstück gebildeten Einlaß sowie einen von einem Rohrstück gebildeten Auslaß aufweist.

Sie zeichnet sich dadurch aus, daß Einlaß und Auslaß an einer gemeinsamen Seite des Reaktors vorgesehen sind, daß eines der beiden Rohrstücke über eine Drehkupplung mit dem Reaktor verbunden ist und daß das andere Rohrstück zumindest im Bereich dieser Drehkupplung im Inneren des einen Rohrstücks angeordnet ist.

EP 0 113 877 A2

/

Einrichtung zur Gewinnung von Energie in Form von Biogas oder
Rottewärme

Die Erfindung bezieht sich auf eine Einrichtung zur Gewinnung von Energie in Form von Biogas oder Rottewärme aus organischen Substanzen, mit einem eine Flüssigkeit aufnehmenden Behälter, mit einem im Innenraum diees Behälters angeordneten Trommelreaktor, der um seine Achse rotierend angetrieben werden kann und einen von einem Rohrstück gebildeten Einlaß sowie einen von einem Rohrstück gebildeten Auslaß aufweist.

Eine derartige Einrichtung ist an sich bekannt (europäische Patentanmeldung 80 10 67 34.9).

Die Einrichtung hat den grundsätzlichen Vorteil, daß sie bei relativ einfacher konstruktiver Ausführen die Gewinnung von Energie in Form von Biogas oder Rottewärme in einem kontinuierlichen Verfahren und bei nahezu optimalen Verhältnissen und hoher Wirtschaftlichkeit gestattet.

Durch die schwimmende Anordnung des Reaktors in dem mit einer Füssigkeit, z.B. mit Wasser gefüllten äußeren Behälter ergeben sich vor allem konstruktive Vorteile hinsichtlich der Ausbildung und Lagerung des Reaktors, d.h. es treten selbst bei großvolumiger Ausbildung des Reaktors keine hohen Kräfte auf, die von der Wandung des Reaktors bzw. von vorhandenen Lagerelementen aufgenommen werden müssen. Weiterhin ergeben sich vor allem auch Vorteile hinsichtlich einer optimalen Wärmeverteilung innerhalb der im Reaktor vorhandenen organischen Substanzen, d.h. insbesondere auch, daß eine optimale Ausnutzung der Substanzen bei der Gewinnung von Energie in Form von Biogas oder Rottewärme erzielt wird.

*2*

Ein weiterer wesentlicher Vorteil dieser Einrichtung besteht darin, daß die Substanzen dem Reaktor kontinuierlich zugeführt und aus diesem Reaktor kontinuierlich abgeführt werden können, so daß ein Arbeiten der Einrichtung ohne verfahrensbedingte Unterbrechungen möglich ist.

Da die für die Gewinnung vo Energie verwendeten Substanzen den trommelartig ausgebildeten Reaktor von einer Seite bzw. Stirnseite zur anderen Seite bzw. Stirnseite durchwandern und somit an einer Stirnseite der eigentliche Einlaß in den Reaktor und an der anderen Stirnseite der eigentliche Auslaß aus dem Reaktor vorgesehen werden muß, ist bei der bekannten Einrichtung an beiden Seiten des Reaktors jeweils ein als Einlaß bzw. als Auslaß dienendes Rohrstück vorgesehen, wobei jedes Rohrstück über eine Drehkupplung mit dem Reaktor verbunden ist. Unter "Drehkupplung" im Sinne der Erfindung ist ein Element zu verstehen, welches eine dichte Verbindung zwischen zwei Teilen herstellt, gleichzeitig jedoch ein Rotieren eines der beiden Teile relativ zum anderen Teil ermöglicht. Zwei derartige Drehkupplungen sind bei der bekannten Einrichtung deswegen erforderlich, weil sich die als Einlaß bzw. Auslaß dienenden Rohrstücke mit dem Reaktor nicht mitdrehen dürfen. Weiterhin ist bei der bekannten Einrichtung nachteilig, daß beide als Einlaß bzw. Auslaß dienenden Rohrstücke bzw. deren Verlängerungen durch die Wandung des Behälters hindurchgeführt sind, wodurch sich Dichtungsprobleme ergeben, und zwar insbesondere dann, wenn die Lagerung des Reaktors nicht ganz exakt ist und sich somit beim Drehen des Reaktors die Achse der Rohrstücke bewegt.

Ein weiterer wesentlicher Nachteil der bekannten Einrichtung besteht noch darin, daß zur Lagerung des Reaktors Lagerböcke Verwendung finden, die am Boden des Behälters befestigt sind, so daß dort ebenfalls Dichtungsprobleme insbesondere dann auftreten, wenn zum Abdichten des Innenraums des Behälters eine Schicht aus wasserundurchlässigem Material vorgesehen ist, die

0113877

J

dann an der Befestigungsstelle der Lagerböcke zwangsläufig unterbrochen werden muß bzw. die beim Montieren der Lagerböcke oder aber während des Betriebs leicht beschädigt werden kann.

Der Erfindung liegt die Aufgabe zugrunde, unter Beibehaltung der grundsätzlichen Vorteile die Nachteile der bekannten Einrichtung zu beseitigen, insbesondere die Anzahl der erforderlichen Drehkupplungen und/oder die Anzahl der erforderlichen Rohrdurchführungen durch die Wandung des Behälters zu verringern sowie generell Dichtungsprobleme insbesondere im Bereich der Innenwandung des Behälters auszuschließen.

Zur Lösung dieser Aufgabe ist eine Einrichtung der eingangs geschilderten Art erfindungsgemäß so ausgeführt, daß der Einlaß und der Auslaß an einer gemeinsamen Seite des Reaktors vorgesehen sind, daß eines der beiden Rohrstücke über eine Drehkupplung mit dem Reaktor verbunden ist und daß das andere Rohrstück zumindest im Bereich dieser Drehkupplung im Inneren des einen Rohrstückes angeordnet ist.

Durch diese Ausbildung ist nur noch eine einzige Drehkupplung erforderlich, wodurch die Kosten für die Herstellung sowie auch vor allem die Kosten für die Wartung der Einrichtung und die Zahl ev. Fehlerquellen wesentlich reduziert werden.

Das zumindest im Bereich der Drehkupplung innere Rohrstück (andere Rohrstück), welches einen kleineren Durchmesser als das äußere Rohrstück aufweist, reicht von der einen Seite des Reaktors bis in den Bereich der anderen Seite dieses Reaktors und mündet dort über eine Öffnung in den Innenraum des Reaktors.

Bei der Erfindung ist der Reaktor vorzugsweise hängend gelagert, d.h. anstelle von Lagerblöcken sind Elemente vorgesehen, die an der Oberseite des Behälters befestigt sind und von oben her in den Innenraum des Behälters hineinreichen und an denen der Reaktor drehbar aufgehängt ist. Diese hängende Lagerung des

4

Reaktors, die von grundsätzlicher Bedeutung ist und auch ohne die besondere Ausbildung des Einlasses sowie Auslasses Vorteile bietet, verhindert vor allem, daß die innere, wasser- bzw. flüssigkeitsundurchlässige Verkleidung des Behälters vor allem in ihrem wirksamen Bereich durch Befestigungselemente usw. unterbrochen werden muß. Durch die hängende Lagerung des Reaktors wird somit das Abdichten des Innenraums des Behälters wesentlich verbessert bzw. vereinfacht.

Weiterbildungen der Erfindung sind Gegenstand der Unteransprüche.

Die Erfindung wird im folgenden anhand der Figuren an einem Ausführungsbeispiel näher erläutert. Es zeigen:

Fig. 1 einen Längsschnitt durch eine Einrichtung gemäß der Erfindung;

Fig. 2 in vergrößerter Detaildarstellung sowie im Schnitt die den Trommelreaktor mit dem äußeren Rohrstück verbindende Drehkupplung;

Fig. 3 einen Schnitt entsprechend der Linie A-B der Fig. 1;

Fig. 4 einen Schnitt entsprechend der Linie C-D der Fig. 1.

In den Figuren ist 1 ein wannen- und rechteckförmiger Behälter, der beispielsweise aus Beton hergestellt ist und einen Boden 2 sowie eine rundherum geschlossene Umfangswand 3 aufweist. An seiner Oberseite ist der dort offene Behälter 1 durch eine oder aber vorzugsweise durch mehrere Deckplatten 4 verschlossen.

An seiner Innenfläche ist der Behälter 1 im Bereich des Bodens 2 sowie im Bereich der Umfangswand 3 mit einer Schicht 5 aus wärmeisolierendem Material ausgekleidet. Auch die Deckplatte bzw. die Deckplatten 4 bestehen zumindest an ihrer dem Innenraum 6 des Behälters 1 zugewendeten Seite aus wärmeisolierendem Material. Als wärmeisolierendes Material eignet sich beispielsweise harter geschäumter Kunststoff. Es sind jedoch auch andere Materialien verwendbar.

Im Bereich des Bodens 2 sowie im Bereich der Umfangswand 3 befindet sich auf der Schicht 5 eine weitere Schicht 7, die aus wasserundurchlässigem Material besteht und die somit die eigentliche Innenfläche des wannenartigen Behälters 1 bildet. Die Schicht 7 ist beispielsweise von einer Kunststoffolie gebildet. Es können auch hier andere Materialien verwendet werden, die zum Abdichten bzw. zur Bildung wasserundurchlässiger Schichten üblich und/oder geeignet sind. Grundsätzlich ist es auch möglich, die Schichten 5 und 7 bzw. die Eigenschaften dieser Schichten (Wärmeisolation und Abdichtung) in einer einzigen Schicht zu integrieren.

Weiterhin ist es grundsätzlich unter bestimmten Voraussetzungen auch möglich, auf die wärmeisolierende Schicht 5 zu verzichten, insbesondere dann, wenn beispielsweise aufgrund der Umgebungsbedingungen eine besondere, wärmeisolierende Schicht 5 nicht erforderlich ist und/oder das für den Boden 2 sowie die Umfangswand 3 verwendete Material eine für den jeweiligen Anwendungsfall genügende Wärmeisolierung bietet und/oder eine wärmeisolierende Schicht entsprechend der Schicht 5 im Boden 2 bzw. in der Umfangswand 3 oder aber an der Außenfläche des Bodens 2 bzw. an der Außenfläche der Umfangswand 3 vorgesehen ist.

Im Innenraum 6 des Behälters 1 ist ein als Trommel ausgebildeter hohlzylinderartiger Reaktor 8 angeordnet, und zwar derart, daß die Trommel- bzw. Symmetrieachse S dieses Reaktors in einer horizontalen Ebene liegt, d.h. in einer Ebene, die parallel zum Boden 2 verläuft.

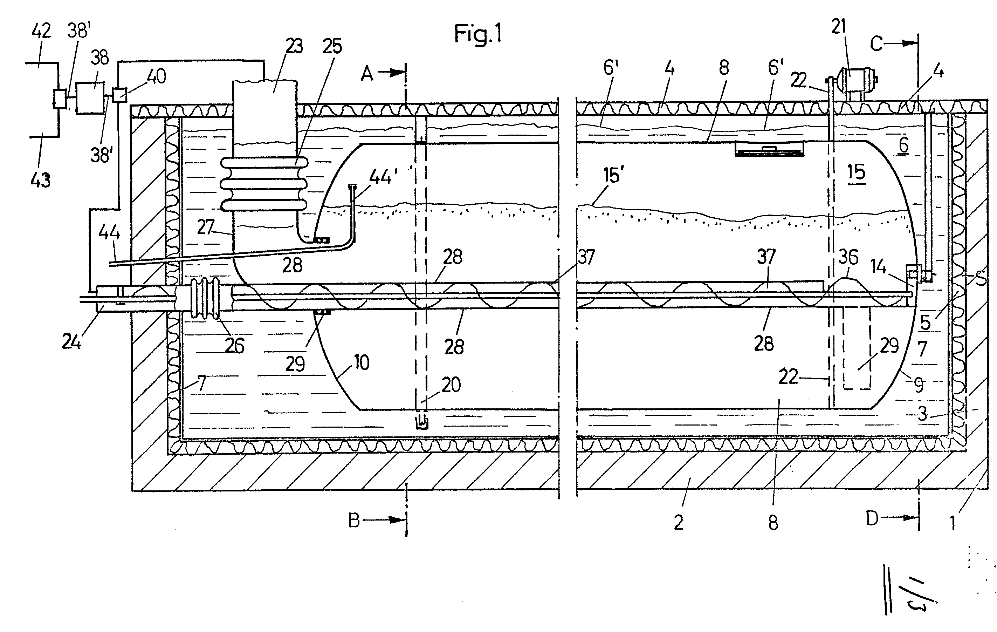

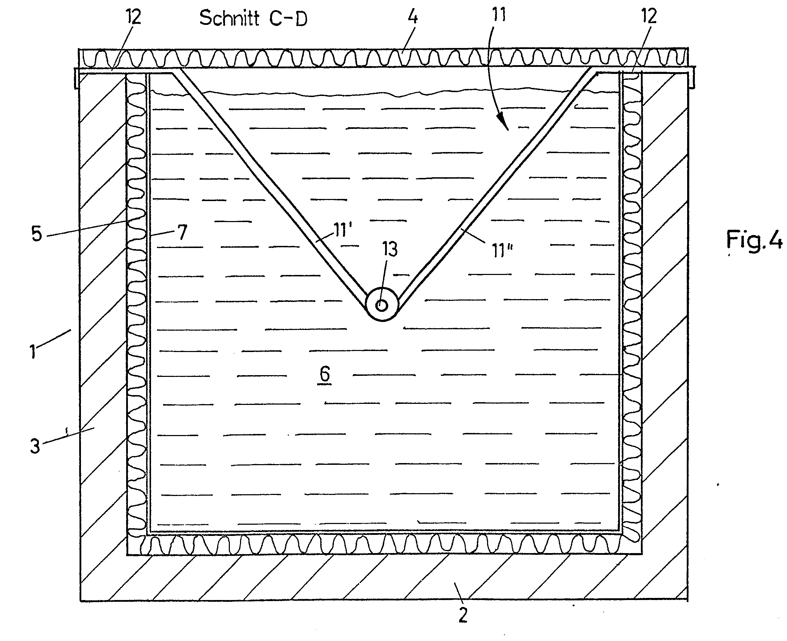

Der an seinen beiden Enden bzw. Stirnseiten 9 und 10 geschlossene und beispielsweise aus Stahl oder aber aus glasfaserverstärktem Kunststoff hergestellte Reaktor 8 ist um seine Symmetrieachse S drehbar gelagert, und zwar im Bereich der Stirnseite 9 an einer bügelartigen Aufhängung 11. Diese Aufhängung besteht bei der dargestellten Ausführungsform aus einem V-förmig abgewinkelten Metallband, welches zwei miteinander verbundene Schenkelarme 11´ und 11´´ bildet, die jeweils an ihren freien, in den Fig. 1 und 4 oberen Enden nach außen gerichtete und abgewinkelte Schenkelabschnitte 12 aufweisen, mit denen die Aufhängung 11 im Bereich der beiden Längsseiten des Behälters 1 auf der Oberkante der Umfangswand 3 aufliegt und dort befestigt ist, wobei die Schenkelabschnitte 12 mit ihren Abwinklungen auch gegen die Außenfläche der Umfangswand 3 anliegen. Die Befestigung der als Lager für den Reaktor 8 dienenden Aufhängung 11 erfolgt somit dort, wo die abdichtende Schicht 7 nicht verhanden ist, d.h. durch die Befestigung der als Lager dienenden Aufhängung 11 wird die abdichtende Schicht 7 an keiner Stelle unterbrochen.

An der Außenfläche der Stirnseite 9 ist am Reaktor 8 ein Lagerzapfen 13 vorgesehen, dessen Achse achsgleich mit der Symmetrieachse S liegt. Zur Halterung dieses Lagerzapfens 13 dient die Stirnseite 9. Am Lagerzapfen ist ein Lagerbock 14 drehbar befestigt, der sich bei der dargestellten Ausführungsform im Innenraum 15 des Reaktors 8 befindet. Es versteht sich, daß der Lagerzapfen 13 abgedichtet durch die Stirnseite 9 hindurchgeführt ist. Das über diese Stirnseite vorstehende Ende des Lagerzapfens 13 liegt auf der Aufhängung 11 im Bereich der Übergangsstelle zwischen den beiden Schenkelarmen 11´ und 11´´ bzw. auf einer dort vorgesehenen Lagerschale, z.B. aus Kunststoff, auf.

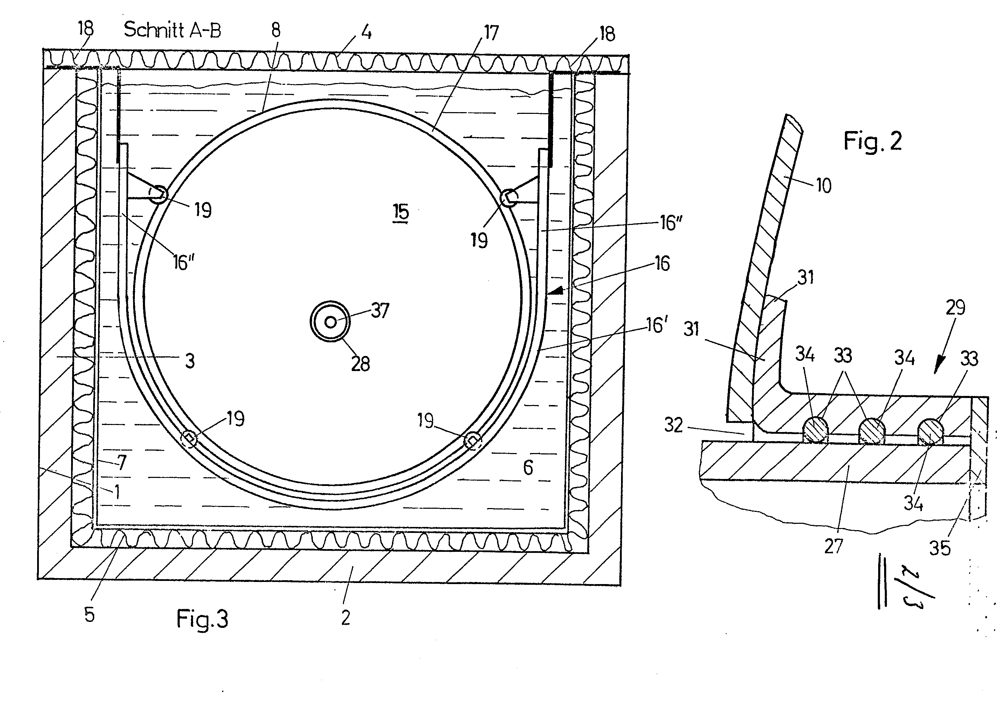

Im Bereich der Stirnseite 10 erfolgt die Lagerung des Reaktors 8 dadurch, daß dort ein den Reaktor 8 teilweise umgebendes Bügelelement 16 vorgesehen ist. Dieses Bügelelement besteht aus einem im wesentlichen kreisförmigen Abschnitt 16´, welcher den jeweils unteren Teil der Umfangswand 17 des Reaktors 8 umgibt, sowie aus zwei Abschnitten 16´´, die die beiden Enden des Bügelelementes 16 bilden und bei der dargestellten Ausführungsform im wesentlichen parallel zur Innenfläche der Umfangswand 3 im Bereich der Längsseiten des Behälters 1 liegen. An den freien, in den Fig. 1 und 3 oberen Enden der Abschnitte 16´´ sind Winkelstücke 18 jeweils mit einem Schenkel befestigt. Der andere Schenkel jedes Winkelstücks 18 ist jeweils an einer Längsseite des Behälters 1 an der dortigen Oberkante der Umfangswand 3 verankert. Am Bügelelement 16 sind mehrere Rollen 19 (bei der dargestellten Ausführungsform insgesamt vier Rollen 19) vorgesehen, gegen die der Reaktor 8 mit der Außenfläche seiner Umfangswand 17 bzw. mit einer dort vorgesehenen, ringförmigen Lagerfläche 20 anliegt.

Wie die Fig. 1 und 3 zeigen, ist auch das Bügelelement 16 dort befestigt, wo die abdichtende Schicht 7 nicht vorhanden ist, d.h. die Lagerung des Reaktors 8 im Bereich der Stirnseite 10 erfolgt wiederum in der Weise, daß eine Unterbrechung der abdichtenden Schicht 7 nicht erforderlich ist.

Es versteht sich, daß sowohl die Aufhängung 11 als auch die von dem Bügelelement 16 gebildete Aufhängung so angeordnet sind, daß diese Aufhängungen bzw. die von den Schenkelarmen 11´ und 11´´ bzw. von den Abschnitten 16´ und 16´´ definierten Ebenen senkrecht zur Symmetrieachse S liegen.

Zum Antrieb des Reaktors 8 und die Achse S dient bei der dargestellen Ausführungsform ein Elektromotor 21, der in geeigneter Weise über dem Innenraum 6 des Behälters 1 befestigt ist und an dessen Welle eine mit einem endlosen Riemen 22 zusammenwirkende Riemenscheibe vorgesehen ist. Der Riemen 22 ist über die Außenfläche der Umfangswand 17 des Reaktors 8 geführt, so daß bei eingeschaltetem Elektromotor 21 der Reaktor 8 über den Riemen 22 rotierend angetrieben wird.

*8*

Wie insbesondere die Fig. 1 zeigt, ist der Innenraum 6 des Behälters 1 bis zu einem Niveau 6´ mit einer Flüssigkeit, vorzugsweise mit Wasser gefüllt, wobei das Niveau 6´ höher liegt als der jeweilige obere Teil der Umfangswand 17 des Behälters 8. Durch die Flüssigkeit im Innenraum 6 ist eine schwimmende Anordnung des Reaktors 8 gewährleistet, wodurch unter anderem sowohl die Lagerung als auch der Antrieb dieses Reaktors vereinfacht werden.

Zum Zuführen der zur Gewinnung von Biogas oder Rottewärme verwendeten Substanzen sowie zum Abführen dieser Substanzen weist die Einrichtung einen Eingang 23 sowie einen Ausgang 24 auf. Beide sind bei der dargestellten Ausführungsform von Rohren gebildet, die jeweils über ein elastisches Rohrstück 25 bzw. 26 mit dem eigentlichen Einlaß bzw. Auslaß des Reaktors 8 in Verbindung stehen. Bei der dargestellten Ausführungsform ist das den Ausgang 24 bildende Rohr durch die Umfangswandung 3 des Behälters 1 sowie durch die Schichten 5 und 7 hindurchgeführt, wobei im Bereich der Schicht 7 sowie im Bereich der Umfangswand 3 für eine ausreichende Abdichtung gesorgt ist. Das den Eingang 23 bildende Rohr ist aus dem Behälter 1 nach oben herausgeführt.

Der eigentliche Auslaß bzw. Einlaß an den Reaktor 8 ist durch zwei Rohrstücke 27 und 28 gebildet, von denen das Rohrstück 27 einen wesentlich größeren Durchmesser als das Rohrstück 28 aufweist. Das Rohrstück 27, welches nach Art eines Krümmers ausgeführt ist, steht mit einem Ende mit dem elastischen Rohrstück 25 in Verbindung und ist mit seinem anderen Ende, d.h. dort wo die Achse des Rohrstückes 27 achsgleich mit der Symmetrieachse S liegt, über eine Drehkupplung 29 an die Stirnseite 10 des Reaktors 8 derart angeschlossen, daß eine abgedichtete Verbindung zwischen dem Rohrstück 27 und der Stirnseite 10 besteht, sich der Reaktor 8 jedoch gleichzeitig relativ zum feststehenden Rohrstück 27 um die Symmetrieachse S drehen kann.

Die Drehkupplung 29 ist im Detail in der Fig. 2 dargestellt. Sie besteht im wesentlichen aus einem Ring 30, der an einem Ende einen nach außen gerichteten Ringflansch 31 besitzt, mit welchem der Ring 30 im Innenraum 15 des Reaktors 8 und im Bereich einer an der Stirnseite 10 vorgesehenen Öffnung 32 derart befestigt werden kann, daß der Ring 30 mit seiner Achse achsgleich mit der Symmetrieachse S liegt. An der Innenfläche besitzt der Ring 30 drei ringförmige Vertiefungen 33, von denen jede einen Dichtungsring 34 teilweise aufnimmt. Das Rohrstück 27 reicht in den Ring 30 hinein, wobei die Dichtungsringe 34 gegen die Außenfläche des Rohrstückes 27 dichtend anliegen. Ein am Ende des Rohrstückes 27 befestigter und über die dortige Umfangsfläche des Rohrstückes 27 vorstehender Ring 35 sichert das Rohrstück 27 gegen unerwünschtes Herausrutschen aus dem Ring 30 der Drehkupplung 29.

Das mit einem Ende mit dem elastischen Rohrstück 26 verbundene Rohrstück 28 ist durch die Wandung des Rohrstückes 27 (im Bereich der Krümmung des Rohrstückes 27) abgedichtet hindurchgeführt, so daß das Rohrstück 28 zumindest im Bereich der Drehkupplung 29 innerhalb des Rohrstückes 27 liegt.

Während das Rohrstück 27 an der Stirnseite 10 in den Innenraum 15 des Reaktors 8 mündet, ist das Rohrstück 28 bis an die andere Stirnseite 9 des Reaktors 8 geführt und besitzt dort eine in den Innenraum 15 mündende Öffnung 36.

Ebenso wie das Rohrstück 27 dreht sich auch das Rohrstück 28, welches an seinem der Stirnseite 9 benachbarten Ende am Lagerbock 24 gehalten ist, nicht mit dem Reaktor 8 mit, d.h. insbesondere, daß eine einzige Drehkupplung 28 ausreicht, um die Verbindung zwischen dem Innenraum 15 des Reaktors 8 und dem Eingang 23 bzw. dem Ausgang 24 herzustellen.

Wie insbesondere die Fig. 1 zeigt, liegt die Achse des Rohrstückes 28 gegenüber der Symmetrieachse S des Reaktors 8 versetzt. Diese Ausbildung ist möglich, wenn der Lagerzapfen 13 an der Stirnseite 9 gehalten ist und der Lagerbock 14 drehbar auf diesem Lagerzapfen 13 sitzt. Selbstverständlich ist es grundsätzlich auch möglich, das Rohrstück 28 so anzuordnen, daß dessen Achse zumindest im Bereich der Drehkupplung 29 und im Innenraum 15 des Reaktors 8 achsgleich mit der Symmetrieachse S liegt. Diese Ausbildung hätte den zusätzlichen Vorteil, daß ev. auch eine sehr einfache zusätzliche Abstützung des Rohrstückes 28 im Bereich zwischen den beiden Stirnseiten 9 und 10 möglich ist. In der Regel ist eine solche zusätzliche Abstützung allerdings nicht erforderlich, da der Innenraum 15 des Reaktors 8 bis zum Niveau 15´ mit den zur Gewinnung von Biogas oder Rottewärme verwendeten Substanzen gefüllt ist und somit eine Art schwimmende Anordnung des Rohrstückes 28 gegeben ist.

Um die verbrauchten Substanzen auszuräumen, d.h. von der Öffnung 36 an den Ausgang 24 zu fördern, ist bei der dargestellten Ausführungsform im Inneren des Rohrstückes 28 eine Förderschnecke 37 angeordnet, die mit ihrem einen Ende am Lagerbock 14 gelagert ist und auch durch das Rohrstück 26 sowie das den Ausgang 24 bildende Rohr reicht. Die Förderschnecke 37 ist durch nicht näher dargestellte Antriebsmittel angetrieben.

Anstelle der Förderschnecke 37 kann auch eine Pumpe 38 verwendet werden, die an den Ausgang 24 angeschlossen ist. In diesem Fall ist die Öffnung 36 vorzugsweise geschlossen und im Bereich der Stirnseite 9 ist am Rohrstück 28 eine nach unten ragende und an ihrem unteren Ende offene rohrförmige Verlängerung 39 vorgesehen, so daß die von der Pumpe 38 angesaugten verbrauchten Substanzen unmittelbar aus dem jeweils unteren Bereich des Innenraumes 15 abgeführt werden. Die Pumpe 38 kann auch gleichzeitig zum Zuführen der Substanzen an den Eingang 23 dienen, was beispielsweise bei einer in ihrer Wirkungsrichtung umschaltbaren Pumpe 38 dadurch möglich ist, daß der eine Anschluß 38´ der

//

Pumpe 38 über einen Wechselschieber 40 wahlweise mit dem Eingang 23 und mit dem Ausgang 24 und der andere Anschluß 38'' der Pumpe 38 über einen Wechselschieber 41 wahlweise mit einer Leitung bzw. einem Anschluß 42 für frische Substanzen und mit einer Leitung bzw. mit einem Anschluß 43 zum Abführen der verbrauchten Substanzen verbunden wird. Durch entsprechende Anschlüsse und durch die Verwendung entsprechender Schieber kann natürlich auch eine nur in eine Richtung fördernde Pumpe 38 sowohl zum Zuführen der Substanzen an den Eingang 23 als auch zum Abführen der Substanzen am Ausgang 24 verwendet werden. In jedem Fall erfolgt die Steuerung der Pumpe 38 zweckmäßigerweise so, daß in einer Arbeitsphase der Pumpe 38 eine bestimmte Menge an Substanzen an den Eingang 23 gefördert wird und in einer darauf folgenden Arbeitsphase der Pumpe 38 die gleiche Menge am Ausgang 24 abgeführt wird, so daß die Gesamtmenge an Substanzen im Innenraum 15 des Reaktors 8 im wesentlichen konstant bleibt.

Bei Verwendung der Einrichtung zur Gewinnung Biogas ist eine Gasleitung 44 vorgesehen, die von außen her abgedichtet durch die Umfangswand 3 des Behälters 1 hindurchgeführt ist und ebenfalls abgedichtet durch die Wandung des Rohrstücks 27 hindurchreicht und durch das mit dem Reaktor 8 verbundene offene Ende des Rohrstücks 27 in den Innenraum 15 des Reaktors mündet, und zwar derart, daß das offene Ende 44' der Gasleitung 44 oberhalb des Niveaus 15' liegt.

Die Arbeitsweise der Einrichtung läßt sich, wie folgt, erläutern:

Es wird davon ausgegangen, daß der Behälter 1 bis zum Niveau 6' mit Wasser und der Reaktor 8 bis zum Niveau 15' mit den organischen Substanzen gefüllt sind.

Für die Gewinnung von Biogas wird das Wasser im Innenraum 6 des Behälters 1 durch nicht näher dargestellte Mittel auf die optimale Betriebstemperatur, z.B. 35 bis 55° C gebracht und auf

dieser optimalen Betriebstemperatur gehalten. Durch entsprechende Erwärmung der Substanzen im Innenraum 15 des Reaktors 8 wird durch vorhandene Bakterien, insbesondere Methanbakterien Biogas erzeugt, welches sich im Raum oberhalb des Niveaus 15´ sammelt und über die Gasleitung 44 abgeführt werden kann. Durch Drehen des Reaktors 8 ergeben sich optimale Verhältnisse hinsichtlich der Temperaturverteilung sowie hinsichtlich der Vermischung der Stoffe im Reaktor 8.

In bestimmten Zeitabständen wird über den Eingang 23 eine bestimmte Menge an Substanzen neu in den Reaktor 8 eingeführt. Eine entsprechende Menge an verbrauchten Substanzen wird über den Ausgang 24 abgeführt.

Bei Erzeugung von Rottewärme (durch einen Fallprozeß bei Anwesenheit von Luft oder Sauerstoff) wird dem Innenraum 15 des Reaktors 8 Luft oder Sauerstoff zugeführt, was grundsätzlich durch die Gasleitung 44 erfolgen könnte oder aber dadurch, daß über den Eingang 23 gleichzeitig auch Luft bzw. Sauerstoff zugeführt wird. Die im Reaktor erzeugte Wärme wird dann an die Flüssigkeit im Innenraum 6 des Behälters 1 abgegeben und kann dort beispielsweise mit Hilfe eines oder mehrerer Wärmeaustauscher entnommen werden.

Die Erfindung wurde voranstehend an einem Ausführungsbeispiel beschrieben. Es versteht sich, daß Änderungen sowie Abwandlungen möglich sind, ohne daß der der Erfindung zugrundeliegende Gedanke verlassen wird.

/

Patentansprüche

1. Einrichtung zur Gewinnung von Energie in Form von Biogas oder Rottewärme aus organischen Substanzen, mit einem eine Flüssigkeit aufnehmenden Behälter, mit einem im Innenraum dieses Behälters angeordneten Trommelreaktor, der um seine Trommelachse rotierend angetrieben werden kann und einen von einem Rohrstück gebildeten Einlaß sowie einen von einem Rohrstück gebildeten Auslaß aufweist, dadurch gekennzeichnet, daß Einlaß und Auslaß an einer gemeinsamen Seite (10) des Reaktors (8) vorgesehen sind, daß eines der beiden Rohrstücke (27, 28) über eine Drehkupplung (29) mit dem Reaktor (8) verbunden ist, und daß das andere Rohrstück (28) zumindest im Bereich dieser Drehkupplung (29) im Inneren des einen Rohrstücks (27) angeordnet ist.

2. Einrichtung nach Anspruch 1, dadurch gekennzeichnet, daß das eine Rohrstück (27) im Bereich der einen, die Drehkupplung (29) aufweisenden Seite (10) in den Innenraum (15) des Reaktors (8) mündet, und daß das andere Rohrstück (28) im Bereich der anderen Seite (9) des Reaktors (8) mit dem Innenraum (15) dieses Reaktors (8) in Verbindung steht.

3. Einrichtung nach Anspruch 1 oder 2, dadurch gekennzeichnet, daß das andere Rohrstück (28) außerhalb des Reaktors (8) abgedichtet durch die Wandung des einen Rohstücks (27) hindurchgeführt ist.

4. Einrichtung nach einem der Ansprüche 1 bis 3, dadurch gekennzeichnet, daß wenigstens ein Rohrstück (27, 28) in seinem außerhalb des Reaktors (8) liegenden Teilbereich als Krümmer ausgeführt ist.

2

5. Einrichtung nach einem der Ansprüche 1 bis 4, dadurch gekennzeichnet, daß das andere Rohrstück (28) an seinem in den Innenraum (15) des Reaktors (8) reichenden Ende an einem an der Innenwandung des Reaktors vorgesehenen Lager (14, 13) gehalten ist.

6. Einrichtung nach einem der Ansprüche 1 bis 5, dadurch gekennzeichnet, daß die Achse des anderen Rohrstücks (28) gegenüber der Drehachse (S) des Reaktors (8) versetzt ist.

7. Einrichtung nach einem der Ansprüche 1 bis 5, dadurch gekennzeichnet, daß die Achse des anderen Rohrstückes (28) achsgleich mit der Drehachse (S) des Reaktors (8) liegt.

8. Einrichtung nach einem der Ansprüche 1 bis 7, gekennzeichnet durch eine Gasleitung (44), die zumindest im Bereich der Drehkupplung (29) ebenfalls im Inneren des einen Rohrstücks (27) verläuft.

9. Einrichtung nach einem der Ansprüche 1 bis 8, dadurch gekennzeichnet, daß zur Lagerung des Reaktors (8) Elemente (11, 16) vorgesehen sind, die an der Oberseite des Behälters (1) befestigt sind und von dort in den Innenraum (6) des Behälters (1) hineinreichen.

10. Einrichtung nach einem der Ansprüche 1 bis 9, gekennzeichnet durch wenigstens eine Pumpe (38) zum Zuführen und Abführen der Substanzen in bzw. aus dem Reaktor (8), vorzugsweise durch eine einzige Pumpe (38), die sowohl zum Zuführen als auch zum Abführen der Substanzen dient.

1/3

Fig.1

Schnitt A-B

Fig.3

Fig. 2

011 3877

Schnitt C–D

Fig.4

0113877